(19) Europäisches Patentamt
European Patent Office
Office européen des brevets

(11) Veröffentlichungsnummer : **0 186 048**
**B1**

(12) # EUROPÄISCHE PATENTSCHRIFT

(45) Veröffentlichungstag der Patentschrift :
01.02.89

(51) Int. Cl.⁴ : **C 07 D303/27, C 07 D301/28**

(21) Anmeldenummer : 85115783.4

(22) Anmeldetag : 11.12.85

(54) Verfahren zur Herstellung von niedermolekularen Glycidylethern ein- und mehrwertiger Phenole.

(30) Priorität : 22.12.84 DE 3447252

(43) Veröffentlichungstag der Anmeldung :
02.07.86 Patentblatt 86/27

(45) Bekanntmachung des Hinweises auf die Patenterteilung : 01.02.89 Patentblatt 89/05

(84) Benannte Vertragsstaaten :
CH DE FR GB IT LI SE

(56) Entgegenhaltungen :
DE–B– 1 103 580
GB–A– 897 744

(73) Patentinhaber : BAYER AG
Konzernverwaltung RP Patentabteilung
D-5090 Leverkusen 1 Bayerwerk (DE)

(72) Erfinder : Prater, Klaus, Dr.
Bodelschwinghstrasse 22
D-4150 Krefeld 1 (DE)
Erfinder : Bottenbruch, Ludwig, Dr.
Wöhlerstrasse 5
D-4150 Krefeld 1 (DE)
Erfinder : Freitag, Dieter, Dr.
Hasenheide 10
D-4150 Krefeld 1 (DE)
Erfinder : Wulff, Claus, Dr.
Richard-Strauss-Strasse 21
D-4150 Krefeld 1 (DE)
Erfinder : Wahle, Bernd, Dr.
Hasselstrasse 35a
D-4044 Kaarst 2 (DE)

EP 0 186 048 B1

## Beschreibung

Die Erfindung betrifft ein verbessertes Verfahren zur Herstellung von Glycidylethern ein- und/oder mehrwertiger Phenole, insbesondere von niedermolekularen Glycidylethern des Bisphenol A.

Es ist bekannt Glycidylether von niedermolekularen ein- und/oder mehrwertigen Phenolen herzustellen indem man zur Lösung der Phenole in überschüssigem Epichlorhydrin, wobei der Überschuß 3 bis 5 Mole Epichlorhydrin pro phenolische Hydroxylgruppe betragen kann, festes Alkalihydroxid oder dessen wäßrige Lösungen zufügt (US-A-2 801 227). Diese Verfahren führt zwar zu den gewünschten Produkten, doch bilden sich durch unerwünschte Nebenreaktionen beträchtliche Mengen an Nebenprodukten (US-PS 2 801 227).

Zum Beispiel aus DE-B-1 103 580 und GB-A-897 744 ist es ebenfalls bekannt, die Reaktion von Phenolen mit Epichlorhydrin in zwei Verfahrensschritte aufzuteilen. Dabei wird in der ersten Stufe Epichlorhydrin in Gegenwart von Katalysatoren, z. B. Salzen quaternärer Ammoniumbasen, z. B. an Bisphenol A zu Bisphenol-bis-chlorhydrinether addiert. In der zweiten Stufe wird dieser Ether mit wäßrigem Alkali zum entsprechenden Bisglycidylether dehydrohalogeniert.

Die Dehydrohalogenierung kann in der Epichlorhydrinlösung erfolgen, wobei die obengenannten Nebenreaktionen ebenfalls ablaufen können. Alternativ kann man das überschüssige Epichlorhydrin abdestillieren, den Rückstand in inerten Lösungsmitteln oder deren Gemischen aufnehmen (z. B. Toluol oder Toluol/Alkohol-Gemische) und mit wäßrigem Alkali die Ringschlußreaktion durchzuführen (DE-B-1 103 580).

Das Verfahren gemäss DE-A-1 103 580 hat zwar den Vorteil, daß Nebenreaktionen bei den angewandten Reaktionstemperaturen in der ersten Reaktionsstufe von 27-40 °C weitgehend vermieden werden können. Auf der anderen Seite bedingen diese Temperaturen aber Reaktionszeiten von 20-96 Stunden, je nach Temperatur und Art des Katalysators. Diese langen Zeiten sind jedoch unwirtschaftlich. Überschreitet man die maximale Reaktionstemperatur von 40 °C, so wird die schwach exotherme Reaktion der Stufe I sehr heftig. Daher kann bei großen technischen Ansätzen die exotherme Reaktion problematisch werden. Außerdem können durch die dann auftretenden Nebenreaktionen Qualität und Ausbeute des Endproduktes stark beeinträchtigt werden.

Es wurde nun gefunden, daß in der ersten Verfahrensstufe eine unkontrollierte exotherme Reaktion, wobei mögliche Nebenreaktionen auftreten können, bei der Umsetzung von Epichlorhydrin mit Phenolen vermieden werden kann, wenn man eine Teilmenge des Epichlorhydrins zusammen mit dem Katalysator vorlegt und dazu eine Lösung des umzusetzenden Phenols in der Restmenge Epichlorhydrin gibt.

Zweistufiges Verfahren zur Herstellung von Glycidylethern ein- und/oder mehrwertiger Phenole, wobei in der ersten Stufe diese Phenole mit Epichlorhydrin in Gegenwart eines basischen Katalysators bei erhöhter Temperatur umgesetzt werden, dadurch gekennzeichnet, daß man die Katalysatoren in einer Teilmenge des Epichlorhydrins löst und auf eine Temperatur von 90-130 °C erhitzt, zu dieser Lösung die auf 20-80 °C erwärmte Lösung der Phenole der restlichen benötigten Teilmenge Epichlorhydrin allmählich zugibt und nach beendetem Eintragen das Reaktionsgemisch 5-10 Stunden bei Siedetemperatur hält.

Anschließend kann das nicht umgesetzte Epichlorhydrin von den gebildeten Chlorhydrinethern abdestilliert werden. Die als Rückstand verbleibenden Chlorhydrinether können in bekannter Weise in einem organischen Lösungsmittel zu den Epoxidharzen dehydrohalogeniert werden.

Die Addition des Epichlorhydrins an Phenole kann bei Temperaturen von 90-130 °C, vorzugsweise bei 110-125 °C, in kurzer Zeit und ohne Nebenreaktionen durchgeführt werden, wenn man zu einer siedenden Lösung des Katalysators in 20-80 Gew.-%, vorzugsweise 30-60 Gew.-%, des insgesamt benötigten Epichlorhydrins eine auf 20-80 °C, vorzugsweise 40-60 °C, erwärmte Lösung des Phenols im restlichen (zu 100 %) Epichlorhydrin so zugibt, daß eine unkontrollierte exotherme Reaktion nicht mehr beobachtet werden kann.

Diese Zeit zur Zugabe kann je nach Größe des Ansatzes unterschiedlich sein. Im allgemeinen beträgt sie 30-90 Minuten. Nach beendetem Eintragen hält man das Reaktionsgemisch noch 5-10 Stunden, vorzugsweise 6-8 Stunden, bei Siedetemperatur. Die Siedetemperatur liegt im allgemeinen bei 120-128 °C.

Die mit Hilfe dieses Verfahrens herstellbaren Epoxidharze zeichnen sich durch einen hohen Gehalt an monomerem Glycidylether des eingesetzten Phenols aus, erhalten in Ausbeuten von 94-98 Gew.-%, bezogen auf das Phenol. Sie besitzen niedrige Viskositäten.

Die Aufarbeitung nach der Umsetzung von Epichlorhydrin mit Phenolen kann in bekannter Weise dadurch erfolgen, daß das überschüssige Epichlorhydrin zusammen mit gebildetem Glycerin-bis-chlorhydrin, gegebenenfalls im Vakuum, abdestilliert.

Zur Dehydrohalogenierung zum Epoxidharz kann der überwiegend z. B. aus Phenol-bis-chlorhydrinether bestehende Rückstand in einem inerten Lösungsmittel, z. B. in Toluol gelöst und zum Sieden erhitzt werden. Zu der siedenden Lösung wird 45 %ige wäßrige Alkalimenge, z. B. Natronlauge zugesetzt, wobei der Anteil 1,2 Mole NaOH pro Mol Cl betragen soll. Die Reaktionszeit beträgt im allgemeinen 5-8 Stunden, vorzugsweise 6 Stunden. Anschliessend wird das Reaktionsgemisch einmal mit Wasser zur Entfernung des gebildeten NaCl gewaschen. Eine zweite Wäsche mit 15 %iger $NaH_2PO_4$-Lösung dient zur Neutrali-

sierung von Restlauge.

Das zuvor abdestillierte Epichlorhydrin wird bei Raumtemperatur mit 45 %iger NaOH gerührt, wobei die Reaktionszeit 5 Stunden beträgt. Die benötigte NaOH-Menge beträgt 10 Gew.-Teile NaOH-Lösung auf 100 Gew.-Teile Epichlorhydrin/Glycerin-bis-chlorhydrin-gemisch. Nach Waschen mit Wasser und NaH$_2$PO$_4$-Lösung, Trennen der Phasen und Andestillieren des Epichlorhydrins bis darin gelöstes Wasser entfernt ist, erhält man ein reines Epichlorhydrin, das in den Prozeß zurückgeführt werden kann. Die Ausbeute an Epichlorhydrin beträgt 95-96 %, bezogen auf die theoretisch zurückgewinnbare Menge.

Als wirksame Katalysatoren werden vorzugsweise quaternäre Ammoniumverbindungen wie z. B. Trimethylbenzylammoniumchlorid, Triethylbenzylammoniumchlorid und Tetrabutylammoniumbromid verwendet. Die Katalysatoren werden in Konzentrationen von 0,01-2 Gew.-%, vorzugsweise 0,1-0,7 Gew.-%, bezogen auf die Menge an Phenolen, eingesetzt.

Als ein- oder mehrwertige Phenole kommen Bisphenole wie z. B. Hydrochinon, Bisphenol A, Bisphenol Z (4,4'-Dihydroxy-diphenylcyclohexan), Tetramethyl-bis-phenol F, Tetrachlorbisphenol A in reiner Form oder aber auch im Gemisch mit einwertigen Phenolen wie z. B. Phenol, Alkylphenolen wie Methylphenol, Oktylphenol und drei- und höherwertigen Phenolen (drei- und mehr OH-Gruppen pro Molekül) wie z. B. Triskresol, wie sie durch Reaktion von Phenolen mit Formaldehyd erhalten werden, zum Einsatz.

Die Menge an Epichlorhydrin beträgt 2,5-10 Mole, bezogen auf eine phenolische Hydroxylgruppe, vorzugsweise jedoch 4-6 Mole. Diese Menge wird zur Durchführung der Reaktion geteilt, wobei ein Teil zur Lösung des Katalysators, der Rest zur Lösung des Phenols verwendet wird. In welchem Verhältnis diese beiden Mengen zueinander stehen, kann von der Löslichkeit des verwendeten Phenols oder Phenolgemisches, das zum Einsatz gelangt, abhängig sein.

Beispiel

Apparatur :
2-l-Vierhalskolben, ausgerüstet mit Rückflußkühler, Tropftrichter, Rührer und Thermometer.

Einwaagen :

a) 462,5 g Epichlorhydrin (5,0 Mole)
   1,15 g Tabac (Triethyl-benzyl-ammoniumchlorid) = 0,5 Gew.-%, bezogen auf Bisphenol A
b) 462,5 g Epichlorhydrin (5,0 Mole)
   228,0 g Bisphenol A (1,0 Mol)

Die Einwaagen unter a) werden vorgelegt, unter Rühren zum Sieden erhitzt und die auf 40 °C erwärmte Lösung b) innerhalb von 40 Minuten zugegeben. Anschließend wird das Reaktionsgemisch 6 Stunden weiter am Rückfluß gehalten. Dabei steigt die Sumpftemperatur auf ca. 125 °C.

Nach beendeter Reaktion wird überschüssiges Epichlorhydrin und gebildetes Glycerin-bis-chlorhydrin im Vakuum bis zu einer Sumpftemperatur von 130 °C abdestilliert. Man erhält 380 g eines hochviskosen Harzes mit folgenden Kennzahlen : Epoxidsauerstoff : 4,3-4,5 %, Chlor : 8,4-8,8 %, Viskosität : 51 000 mPas (Hoeppler 25 °C). Der Rückstand wird in 400 ml Toluol aufgenommen, auf Rückflußtemperatur erhitzt und innerhalb von 30 Minuten 98,1 g NaOH 45 %ig zugegeben. Man läßt 6 Stunden nachreagieren, wobei sich die Sumpftemperatur auf ca. 98 °C einpendelt. Nach dem Abkühlen auf Raumtemperatur wird zunächst mit 400 ml Wasser, dann mit 200 ml 15 %iger NaH$_2$PO$_4$-Lösung gewaschen. Der pH sollte danach ≤ 6 sein. Nach dem Abtrennen der wäßrigen Phase wird das Lösungsmittel abdestilliert ; das Sumpfprodukt wird während 3 Stunden bei einer Temperatur von 125-130 °C mit einem kräftigen Stickstoffstrom ausgeblasen. Anschließend wird der Rückstand über eine Filterschicht (KoOO-Filter der Fa. Seitz) gedrückt.

Epoxidäquivalent : 180-183
Restchlor : 0,4-0,5 %
Viskosität : 8 400 mPas (Hoeppler 25 °C)
Ausbeute : 98 %, bezogen auf Bisphenol A

**Patentansprüche**

1. Zweistufiges Verfahren zur Herstellung von Glycidylethern ein- und/oder mehrwertiger Phenole, wobei in der ersten Stufe diese Phenole mit Epichlorhydrin in Gegenwart eines basischen Katalysators bei erhöhter Temperatur umgesetzt werden, dadurch gekennzeichnet, daß man die Katalysatoren in einer Teilmenge des Epichlorhydrins löst und auf eine Temperatur von 90-130 °C erhitzt, zu dieser Lösung die auf 20-80 °C erwärmte Lösung der Phenole der restlichen benötigten Teilmenge Epichlorhydrin allmählich zugibt und nach beendetem Eintragen das Reaktionsgemisch 5-10 Stunden bei Siedetemperatur hält.

2. Verfahren nach Anspruch 1, dadurch gekennzeichnet, daß als Katalysatoren Salze quaternärer Ammonium- und Phosphoniumbasen verwendet werden.

3. Verfahren nach Anspruch 1, dadurch gekennzeichnet, daß die Teilmenge zur Lösung des basischen Katalysators 20-80 Gew.-% der insgesamt eingesetzten Epichlorhydrinmenge beträgt.

4. Verfahren nach Anspruch 1, dadurch gekennzeichnet, daß die gesamt eingesetzte Epichlorhydrinmenge 2,5-10 Mole/phenolische Hydroxylgruppe beträgt.

5. Verfahren nach Anspruch 1, dadurch gekennzeichnet, daß die Menge an basischem Katalysator 0,01-2 Gew.-%, bezogen auf die Gesamtmenge an Phenolen, beträgt.

**Claims**

1. Two-stage process for the preparation of glycidyl ethers of monovalent and/or polyvalent phenols, in which in the first stage, these phenols are reacted with epichlorohydrin at an elevated temperature in the presence of a basic catalyst, characterised in that the catalysts are dissolved in a portion of the epichlorohydrin and heated to a temperature of 90 to 130 °C and a solution of the phenols heated to 20 to 80 °C the remainder of the required quantity of epichlorohydrin is gradually added to the first mentioned solution and the reaction mixture is kept at the boiling temperature for 5 to 10 hours after all the solution has been added.

2. Process according to claim 1, characterised in that the catalysts used are salts of quaternary ammonium and phosphonium bases.

3. Process according to claim 1, characterised in that the portion of epichlorohydrin used for dissolving the basic catalyst amounts to 20 to 80 % by weight of the total quantity of epichlorohydrin put into the process.

4. Process according to claim 1, characterised in that the total quantity of epichlorohydrin put into the process amounts to 2.5 to 10 mol/phenolic hydroxyl group.

5. Process according to claim 1, characterised in that the quantity of basic catalyst amounts to 0.01 to 2 % by weight, based on the total quantity of phenols.

## Revendications

1. Procédé en deux étapes pour produire des éthers glycidyliques de monophénols et/ou de polyphénols, dans lequel, dans la première étape, on fait réagir à température élevée ces phénols avec l'épichlorhydrine en présence d'un catalyseur basique, procédé caractérisé en ce qu'on dissout les catalyseurs dans une quantité partielle de l'épichlorhydrine et l'on chauffe la solution à une température de 90-130 °C, on ajoute progressivement à cette solution la solution, chauffée à 20-80 °C, des phénols dans la quantité partielle restante nécessaire d'épichlorhydrine et, après achèvement de l'introduction, on maintient le mélange réactionnel à la température de l'ébullition durant 5 à 10 heures.

2. Procédé selon la revendication 1, caractérisé en ce qu'on utilise comme catalyseurs des sels de bases d'ammonium quaternaire et de phosphonium quaternaire.

3. Procédé selon la revendication 1, caractérisé en ce que la quantité partielle utilisée pour dissoudre le catalyseur basique représente 20 à 80 % en poids de la quantité totale d'épichlorhydrine que l'on utilise.

4. Procédé selon la revendication 1, caractérisé en ce que la quantité totale d'épichlorhydrine que l'on utilise représente 2,5 à 10 moles par groupe hydroxyle phénolique.

5. Procédé selon la revendication 1, caractérisé en ce que la quantité du catalyseur basique représente 0,01 à 2 % en poids, par rapport à la quantité totale des phénols.